# EUROPEAN PATENT APPLICATION

(11) **EP 2 548 572 A2**
(43) Date of publication of application: **23.01.2013**
(21) Application number: 11777295.4
(22) Date of filing: 05.05.2011
(51) Int. Cl.: A61K 39/015, A61P 33/06

(54) **ADJUVANTED TOLEROGENS AS A MALARIA VACCINE**

(30) Priority: 05.05.2010 CU 20100083
(71) Applicant: Instituto Finlay, Centro de Investigacion-Produccion de vacunas y sueros, La Coronela, La Lisa, Ciudad Habana 11600 (CU)
(72) Inventor: PÉREZ MARTÍN, Oliver Germán, C. Habana, 11300 La Habana (CU); PÉREZ CUELLO, Maribel, C. Habana, 11600 La Habana (CU); CABRERA BLANCO, Osmir, C. Habana, 11600 La Habana (CU); BALBOA GONZÁLEZ, Julio Antonio, C. Habana, 10600 La Habana (CU); LASTRE GONZÁLEZ, Miriam de San Juan Bosco, C. Habana, La Habana 11300 (CU); ZAYAS VIGNIER, Caridad, Caridad, C. Habana, La Habana 11600 (CU); GONZÁLEZ AZNAR, Elizabeth, C. Habana, 10600 La Habana (CU); ROMEU ALVAREZ, Belkis, C. Habana, 19200 La Habana (CU)
(74) Representative: Jansen, Cornelis Marinus
(86) International application number: PCT/CU2011/000002
(87) International publication number: WO 2011/137876

(57) **Abstract**

The invention relates to the field of vaccinal compositions for the effective or preventive treatment of vertically transmitted infections, especially malaria and preferably human malaria caused by *Plasmodium falciparum.* The technical aim of the invention is to use the tolerogens transferred, at an early stage, from the mother to the foetus/newborn and to formulate them with potent adjuvants that are inducers of Th1 and cytotoxic T-cell responses in order to use same in prophylactic or therapeutic vaccines against vertically transmitted infections, especially malaria. The use of said formulations at early ages via the parenteral route or via the mucosal route or both at the same time reverts the tolerance induced by the transfer of tolerogens, thereby inducing protective responses.

## Description

This invention is related to the field of vaccines, specifically the production and use of adjuvant vaccines in the treatment or prevention of vertically transmitted infections and particularly malaria.

All study materials and information disclosed in publications and patents or patent applications mentioned in these specifications are included here for reference purposes (EP 885900077.8 or US 5, 597,572; WO/2004/047805; WO/2003/094964, EP1716866, CU/P/2008/215; Pérez O et al. Infect Immun. 2001, 69(7):4502-4508; Pérez O et al. Immunology and Cell Biology. 2004, 82:603-610; Rodriguez, T et al. Vaccine 2005, 26:1312-21; Pérez O, et al. Scand J Immunology 2007, 66:271-77).

**The technical aim** of the invention is to use tolerogens transferred at an early stage from mother to foetus/newborn and to formulate them with potent adjuvants, particularly derivatives of *Neisseria meningitidis* serum group B (AFPL1, Adjuvant Finlay Proteoliposome 1 and AFCo (cochleate)), inducers of Th1 and CTL responses for use in prophylactic or therapeutic vaccines against vertically transmitted infections, especially malaria. The use of these formulations at early ages reveres the tolerance inducing protective responses.

### Current status

The WHO declares malaria as the most significant tropical illness, which kills more individuals than any other transmissible disease except for acquired immunodeficiency syndrome (AIDS) and tuberculosis. It estimates that there are between 350 and 500 million annual cases of clinical malaria, resulting in 1.1 million deaths per year. The highest rate of neonatal death occurs in children under 5 years. Malaria is endemic in some 100 countries which are home to around 40% of the world's population (WHO/IVB/06.01:46-55, 2006). It is estimated that a child dies of malaria in endemic countries every 30 seconds.

Malaria is caused by protozoans of the genus *Plasmodium.* There are around 156 species of this genus which infect different vertebrates. Among these, *P. berghei, P. chaboie* and *P. yoelii* infect rodents; *P. knowlesi* and *P. cynomolgi* infect non-human primates, and just 5 species - *P*. *falciparum, P. vivax, P. ovale* and *P. malarias* and more recently *P. knowlesi* - infect humans.

Infection is caused by females of the *Anopheles* genus of mosquito which need blood to reproduce. There are known to be around 50 species of *Anopheles* which act as intermediate hosts. Despite this and the restriction of the host, the cycle is very similar. The mosquito inoculates the host with the infective sporozoites form of the disease found in its saliva and, once in the bloodstream, it takes 30 minutes to reach the liver and penetrate the hepatocytes. Each hepatocyte is infected by a single sporozoite, where through asexual schizogony it transforms into schizonts which in turn give way to tens of thousands of merozoites in just 6-16 days. The ruptured hepatocyte flows into the bloodstream and invades the haematites. Multiplication and maturation then occur in 24 to 72 hours, depending on the species. Each erythrocyte explosion is the consequence of a parasitic multiplication and leads to the invasion of new erythrocytes. The typical signs of malaria, such as acute fever and stiffness occurring every 48-72 hours, coincide with the synchronised lysis of the infected erythrocytes. Some merozoites transform into the sexual stage, gametocytes, which are picked up by the mosquito where they are bound and evolve into sporozoites.

Signs/symptoms of malaria include: high fever, malaise, headache, joint pain, nausea and vomiting. *P. falciparum* infection is more severe due to the ability of infected haematites to adhere to the blood vessels. Acute renal failure, cerebral malaria and pulmonary oedema occur more commonly in non-immune populations such as children and travellers from non-endemic countries. Pregnant women are another high-risk group; malaria causes severe anaemia, which is the primary cause of maternal death during pregnancy. Concomitant infection with human immunodeficiency virus (HIV) and malaria in pregnant mothers propitiates infection of the newborn with HIV (Miller LH, et al. Nature, 2002, 415:673-679 and WHO/TVB/06.01:46-55, 2006).

In regions where vector control using Abate has failed, chemotherapy, consisting of a limited selection of anti-malaria agents, is the only line of defence against the disease. The increased resistance to medications in all endemic malaria regions is significant cause for concern and has prompted calls for the development of new anti-malaria measures, which will include a variety of drugs, as well as a broad spectrum of vaccination strategies (Richie TL and Saúl A. Nature 2002, 415:694-701, Gardner MJ et al. Nature 2002, 419:498-511 and Banyal HS and Inselburg J. Am. J. Trop. Med. Hyg., 1985, 34:1055-1064).

In the fight against malaria, great efforts have been made by multiple organisations to improve both diagnosis and chemotherapy, as well as implementing control measures in the form of insecticide-soaked mosquito nets and residual insecticide fumigation. However, the emergence or resurgence of countries with malaria is evident, which makes the development of a safe, effective vaccine for general use a top priority in terms of global public health (WHO/IVB/06.01:46-55, 2006).

The term "premunition" was introduced to define immunity against malaria characterised by the concomitant condition of resistance with the persistence of parasites (Sergent ED, Parrot L, Institute Pasteur d'Algérie Archives, 1935, 3:279-319). This only occurs in hyperendemic countries after at least two decades continuous exposure (Gordon- Thomson J, Immunity in Malaria. Trans. Roy. Soc. Trop. Med. Hyg. 1933, 26(6):483-514). Premunition, or non-sterilising immunity, is acquired very slowly, causing instability and making it impossible to eliminate the infection, which disappears due to a lack of reinfection for more than one year.

The effector immune response is generally split in half by antibodies or cells. This division is often incorrectly interpreted, the assumption being that the former derives from a Th2 pattern and the latter from Th1. What is not taken into account is that the Th1 response also produces a good functional antibody response (complement fixation and with opsonophagocytosis [cytophilic] capacity) and induces good memory response. Moreover, the induction of a CTL response and its duration (memory) only occurs when a potent Th1 response is established. If we take these considerations into account, assuming that the antibody or cell response is responsible for protecting against any of the stages of the malaria cycle tends to result in confusion regarding their nature.

The marginalisation of infected erythrocytes and gametocytes is one of the parasite escape mechanisms fundamentally intended to prevent splenic elimination in the former and to facilitate uptake of the latter by mosquitoes. And although this marginalisation occurs in many organisms, it is particularly important in the placenta during pregnancy, when the merozoites change their vascular adhesion receptors from CD36 to chondroitin sulphate A (CSA). It is thought that merozoites from the placenta are different to their circulatory variant and that, in the placenta, this variant remains independent of the other.

The influence of the presence or absence of immunity to malaria in pregnant women is another serious problem during pregnancy, it being known that non-immune pregnant women, followed by those who are semi-immune, are more affected by malaria than pregnant women who are immune. On the other hand, parity (number of childbirths) is a determining factor, with primigravidae and, therefore, young mothers being the most affected. This is due to the fact that during successive pregnancies, the mother comes into contact with new variants of parasites in the placenta and acquires a certain degree of immunity to them. However, this contradicts the need for at least two decades of exposure to acquire immunity, which itself is lost in just one year with no exposure to infected mosquitoes. This binding of parasites in the placenta produces an increase in: death of both mother and newborn; miscarriage; maternal and baby anaemia, stillborns and low birth weight.

There is evidence that a prophylactic malaria vaccine can be obtained, such as: 90% protection of immunised individuals with irradiated sporozoites; acquiring 'premunition' after decades of continuous exposure in highly endemic areas, and protection through the passive transfer of specific antibodies (WHO/IVB/06.01:46-55, 2006).

In humans there are two important populations which require different approaches: inhabitants of endemic areas and travellers who have never been in contact with malaria. Among the former, there are various at-risk groups subject to repeated infections with *P. falciparum*: a) children under one year of age, especially those suffering from life-threatening anaemia; b) children aged 2 to 5 who suffer from induced coma (Marsh K and Snow RW. Parasitología 1999, 41:241-246); and c) primigravidae suffering from severe illnesses related to the binding of parasites in the placenta (Ricke CH et al. J. Immunol. 2000, 165:3309-3316), especially non-immune primigravidae.

**For expectant mothers:** Reappearance of anaemia; maternal morbidity/death and high rates of miscarriage (Heard D and Jordan T. Cent. Afr. J. Med., 1981, 27:62-68), as well as stillborns and low birth weights, are frequent occurrences with malaria. The increased severity of malaria during pregnancy, especially in primigravidae (McGregor IA. Am. J. Trop. Med. Hyg., 1984, 33:517-25 and Brabin A. Int. J. Epidemiol., 1991, 20:276-83), is a widely accepted fact. However, its mechanisms are not entirely clear. As such, there are various theories which try to partially explain this phenomenon:
- Firstly, the Th1 immune response required for protection against malaria (Fried M, et al. J. Immunol., 1998, 160:2523-30, Fievet N, et al. J. Infect. Dis., 2001, 183:1530-34 and Suguitan AJL et al. J. Infect. Dis., 2003, 188:1074-82) is depressed during pregnancy (Riley EM, et al. Am. J. Trop. Med. Hyg., 1989, 40:141-4, Fievet N, et al. Am. J. Trop. Med. Hyg., 1995, 53:612-17 and Recusen EN, et al. Am. J. Reprod. Immunol., 2001, 46:169-79), since pregnancy generates a systemic Th2 response to protect a foetus incompatible with the mother due to paternal influence from being rejected. This response, along with exacerbation of the anaemia, could be one of the causes of miscarriage or early foetal death.
- Secondly, anti-adhesion antibodies against CSA-binding parasites are associated with protection and are acquired over successive pregnancies (Ricke CH et al. J. Immunol., 2000, 165:3309-16, O'Neil-Dunne I, et al. Infect. Immun., 2001, 69:7487-92, Fried M and Duffy PE. Science 1996, 272:1502-1504). During infection, merozoites in peripheral blood bind to vascular endothelia through CD36, while those captured in the placenta are bound to CSA (Fried M y Duffy PE. Science, 1996, 272:1502-1504, Beeson JG, et al. Nat. Med., 2000, 6:86-90), both representing clones with different variant surface antigens (VSA) (Ofori MF, et al. Infect. Immun., 2003, 71(3):1584-1586). Several families of VSA-encoding genes have been identified. The best characterised is the var gene family, which encodes the erythrocyte membrane protein 1 of *P. falciparum* (PfEMPl) (Smith JD, et al. Cell, 1995, 82,101-110, Baruch DI, et al. Cell, 1995, 82,77-87 and Su X, et al. Cell, 1995, 82:89-100). Two var genes which are less polymorphic than the rest of the var family, var1CSA and var2CSA (Salanti A, et al. Mol. Microbiol., 2003, 49;179-191), have been identified and are seen as promising vaccine candidates. However, any vaccine of this type will not protect against infection, since the response observed combats erythrocyte stages but not the hepatic or sexual stages. There is also the possibility of outbreak through mutants in these genes which evade immunity and since the sequences var1CSA and var2CSA vary between strains, so an effective vaccine would have to stimulate a widespread antibody response. This theory would also fail to explain why mothers who produce antibodies against CSA-binding parasites from the second trimester of pregnancy are not protected (Ricke CH, et al. J. Immunol., 2000, 165:3309-3316 and Staalsoe T, et al. J. lnf. Dis., 2001, 184:618-626).
- Thirdly, non-specific immune suppression due to pregnancy-related hormones which depress innate response, especially natural killer (NK) cells (Bouyou-Akotet MK, et al. CID, 2004, 38:342-347).

**For the newborn:** Death due to miscarriage; stillborns; anaemia, prematurity and low birth weight are frequent occurrences caused by the capture of parasites in the placenta (Desowitz RS, et al. Am J Trop Med Hyg, 1989, 41(6):630-4). For children born successfully, the influence of maternal malaria infection could be positive or negative:
- **On the positive side**, in humans there is the transfer of anti-malaria antibodies (IgG or IgA) through the placenta and/or breast milk which protect the newborn during his first few months of life, and the transfer of antigens to stimulate priming at the foetal or perinatal level.
- **On the negative side**, it can be assumed that the transfer of antigens into an immature immune system induces tolerance; that an inappropriate response (Th2) is induced; or that the antibodies interfere with future response to infection by affecting the fine specificity of the immune response (Stanisic DI, et al. J Immunology 2003, 172:5570-5581).

Murines are infected by species such as *P. chaboudi; P. vinckei, P. yoelii* or *P. bergei* within which there are infectious strains with varying degrees of lethality and which can even reproduce cerebral malaria. The strain *P. bergei* NK65 is one of the most lethal, producing death in mice in less than 20 days of inoculation.

On the one hand there are reports of infection in murines (mice and rats) with *P. bergei* NK65 during gestation (Leopardo O, et al. Patológica, 1994, 86(3):284-290, Desowitz RS, et al. Am. J. Trop. Med. Hyg., 1989, 41(6):630-634, Oduela AM, et al. J. Protozool., 1982, 29(1):77-81 and Hioki A, et al. J. Protozool., 1990, 37(3):163-167) which, depending on the days of inoculation, manage to complete the gestation period. These works were carried out prior to 1995 and, while confirming that the model is possible, they do not benefit from the recent immunological findings related to innatist theories and antigen and regulatory T-cell processing and presentation, which are revolutionising approaches to immune response induction and regulation. There is of course a resurgent need for infection models during gestation to test anti-malaria vaccines (Galinski MR and V Corredor. Mol. Biochem. Parasitol., 2004, 134:17-25) and works are already emerging on suppressor T-cells during infection with *P. berghei* NK65. This study on the induction of protection was conducted on adult mice (Long TT, et al. Int. J. Parasitol., 2003, 33(2):175-83).

On the other hand, the transfer of antigens and even parasites from mother to offspring is not uncommon in either murine or human malaria, although the transfer rates are not completely clear.
- Studies on immune female mice induced by repeated anti-malaria infections/treatments have revealed a transfer of cells and antibodies which affect the development of the subsequent vaccine and even the behaviour of the malaria infection two weeks after birth (Stanisic DI, et al. J. Immunology, 2003, 171:5461-5469 and Stanisic DI, et al. J Immunology, 2003, 172:5570-5581). In pregnant women having acquired malaria in the placenta, the transfer of parasites to the newborn can vary with reported rates of up to 50%. However, parasites are thought to be transferred at low concentrations and do not result in congenital malaria (Ismaili J, et al. Clin. Exp. Immunol., 2003, 133:414-421).

Nonetheless, no murine studies have looked at the early infection of offspring of infected mothers under the influence of a potential perinatal transfer of parasites (Akingbade OA. J. Reprod. Med., 1992, 37(3):273-6) or antigens (Guoling Xi, et al. Infec. Immun., 2003, 71:1242-46), and it is known that placenta uptake produces severe alterations and can even induce necroses in the placenta (Desowitz RS, et al. Am J Trop Med Hyg, 1989, 41(6):630-4).

In humans, high exoantigen levels have been found in umbilical cord serum (Iakodsen PH, et al. Immunology, 1998, 93:264-9) with the risk of the newborn developing congenital malaria dependent upon immune status and uptake of malaria in the mother's placenta. This risk ranges from: very high in primigravidae or non-immune multigravidae (non-endemic areas); high in semi-immune primigravidae (areas of low endemicity); moderate in semi-immune multigravidae (areas of low endemicity); low in hyperimmune primigravidae (hyperendemic areas); to very low in hyperimmune multigravidae (hyperendemic areas) (Hviid L and Staalsoe T. Trens in Parasitology, 2004, 20(4):66-72). This risk does not include the transfer of antigens or anti-idiotypic (ID)/anti-ID antibodies which resemble antigens (Rajewsky K y Takemori T. Ann. Rev. Immunol., 1983, 1:569 and Kaerney JF, et al. Chem. Immunol., 1990, 48:1). This risk classification has been associated with an approach involving changes in *P*. *falciparum* sub-clones which express different VSAs: VSASM (severe malaria); VSAUM (uncomplicated malaria) and VSAPAM (pregnancy-associated malaria) (Smith JD y Deitsch KW. J. Exp. Med., 2004, 200(9):1093-1097 and Rajewsky K and Takemori T. Ann. Rev. Immunol., 1983, 1:569) which therefore escape pre-existing antibodies. This parasitological/humoral approach focuses on the pressure on antibodies to induce changes in parasites through VSA variation. However, no consideration is given to either cell response (Th1 and CTL), which is vital in protecting against malaria and ultimately produces the desired antibodies (as mentioned above), or the influence of possible malaria antigens which reach the foetus/newborn in an immature immunological environment and under the influence of cytokines and/or suppressor T-cells.

It should be remembered that during both murine and human gestation there are hormonal and immunological changes which promote the switch from a Th1 pattern to Th2, and that the newborn also has a greater production of Th2 cytokines than Th1 (Hassan J and Reen DJ. Immunology Today, 2000, 21:107-8). The newborn has an immature immune system where the functions of dendritic cells, professional presenting cells, are diminished with a subsequent deficit in the production of both IL-12 (Goriely S et al. J. Immunol., 2001, 166:2141 -6), a cytokine involved in the guidance towards a Th1 pattern, and IFND which are required to control malaria infection. *P. falciparum* also inhibits the maturation of dendritic cells (Urban BC, et al. Nature, 1999, 400:71-73). The newborn also has neutropenia and is deficient in certain complement components which could also be implicated. There have been reports of a production of suppressor cytokines such as IL-10 and TGFB through the infected placenta together with suppressor T-cells (Suguitan AL, et al. Amer. J Trop. Med. Hyg., 2003, 69:574-81 and Stanisic DI, et al. J Immunology 2003, 171:5461-5469). IL-10, anaemia, low TNFa/IL-10 ratio and high parasitaemia are risk factors for the mother-foetus relationship (Tilg H, et al. J Immunol 2002, 169:2204-2209) while IL-10 has also been associated with a rise in anaemia (Tilg H, et al. J. Immunol., 2002, 169:2204-2209 and Jeans RT Jr. Curr. Hematol. Rep., 2003, 2:116-121). For this reason, the possible transfer of antigens through excretion/secretion or parasites in a suppressor micro-environment can induce tolerance or an inadequate response to certain antigens that are essential to protect against malaria.

Protection against congenital malaria in newborns can occur through:
1. Antibodies derived from the mother, transferred through the placenta (Edozien JC, et al. Lancet, 1962, 2:951; Akanmori BD, et al. Trans. R. Soc. Trop. Med. Hyg., 1995, 89:560, Campell CC, et al. Am. J. Trop. Med. Hyg., 1980, 29:151, How B, et al. Infect. Immun., 1995, 63:4034, Branco OH, et al. J. Infec. Dis. 2000, 181:1746, Bruce-Chwatt I, Nature, 1954, 173:353 and Ferry RJ. Trans. R Soc. Trop. Med. Hyg. 1956, 50:41). In murines, it is hypothesised that this takes place essentially through milk (Halliday R. Proc. R. Soc. London. Ser., 1955, B 144:427). However, we have discovered transfer through the placenta;
2. Presence of foetal haemoglobin which makes erythrocytes less susceptible to infection (Pasvol G, et al. Nature, 1977, 270:171);
3. Absence of para-aminobenzoic acid required by the parasite in maternal milk (Hawking F. Br. Med. J., 1954, 1:1201 and Maegraith BG, et al. Br. Med. J., 1952, 2:1382) and
4. Reduced exposure to mosquito bites (MacDonald G. Trop. Dis. Bull., 1950, 47:915) using mosquito nets, in some cases soaked with insecticides.

This protection could explain why children under 6 months are relatively protected against severe malaria and its associated death rate and high asexual parasitaemia (McGuinness d, et al. Trop. R. Soc. Trop. Med. Hyg., 1998, 92:527). However, malaria involves a single occurrence of parasite uptake in the placenta which can result in foetal death, prematurity, low birth weight and stillborns. Additionally, significant alterations in placental architecture can promote the transfer of antigens or parasites which can be sufficiently eliminated in the presence of maternal antibodies. Formed immune complexes (Desowitz RS. Am. Trop. Med. Parasitol., 1988, 82:121, Jacoksen PH, et al. Immunology, 1998, 93:264-269 and Kasuhara Y, et al. Int. J. Parasitol., 2000, 24:609-15) could also promote response in an unfavourable micro-environment. There are reports that these antigens produce T-cell priming in newborns (Rasheed FN, et al. Parasite. Immunol. 1995, 17:1 and Fievet N, et al. Parasite. Immunol., 1996, 18:483, Jacoksen PH, et al. Immunology, 1998, 93:264-269). If this occurs, it is to be expected that this stimulation will be of Th2 lymphocytes and not Th1 protectors. Evidence which points to this is the production of IL-4 as well as 1FN□ following sensitisation in the uterus (King CH, et al. J. Immunol., 2002, 168:356-364), production of anti-malaria IgM and IgE in newborn serum (Achidi EA and Salimanu LS, Afr. J. Med. Sci., 1997, 26:167-70, Chizzolini C, et al. Am. J. Trop. Med. Hyg., 1991, 45:57-64, Deloron P, et al. Clin. Exp. Immunol., 1997, 110:212-218, Desowitz RS et al. PNG Med. J., 1992, 35:303-305 and Egwunyenga OA, et al. J. Commun. Dis. 1995, 27:77-83), an increase in sCD30 and IL13 (Holt PG, et al. Allergy 2000, 55:688-97) and a predominance of IgGl in mice (Malhotra I, et al. J. Immunol., 2001, 166:2141-6). It should be remembered that IL-4, IgE, sCD30, IL-13 and, in mice, IgGl, are characteristic of a Th2 response.

It is known that allergens capable of passing through the placental barrier (Holt PG, et al. Allergy, 2000, 55:688-97) and, along with helminths, are the principal inducers of IgE and of an Th2 pattern. It is also known that the response to BCG immunisation is depressed in children of mothers with helminthiasis (Malhotra I, et al. J. Immunol., 2001, 166:2141-6). While the appearance and development of allergies is related to factors such as environment, their basic role in an individual's propensity to develop a Th2/IgE response to environmental antigens is genetic (Holgate ST. J. Allergy Clin. Immunol., 1999, 104:1139-1146). Consequently, descendants of allergic parents will also be allergic with a greater frequency than children of non-allergic parents (this frequency is estimated to be around 75, 50 and 25% if both parents, the mother or the father are allergic, respectively). This means there is a high probability of predicting the behaviour of an individual within the population and, therefore, whether there is a system of detection at the parental level. There is also known to be a strengthening of the Th2 pathological pattern during the child's first 6-24 months, while environmental factors such as exposure to bacterial infections can positively influence the disease inducing a Th1 pattern, which counter-regulates allergic Th2 (Holt PG. Allergy Clin. Immunol. International, 2000, Sl:83-85). These findings allude to the possibility of designing prophylactic anti-allergic vaccines, involving the strengthening of the Th2 pattern towards allergens in early infancy. Such vaccines could be based on the use of Th1 adjuvants formulated with allergens found to be effective during lactation.

Chloroquine has been and is still used as an anti-malaria agent. It is an immune-modulating drug (Ertel W, et al. Blood, 1991, 78:1781-8 and Landewe R, et al. J. Rheumatol. 1992, 19:1353-7) which, when used in chemoprophylaxis or treatment during pregnancy, could increase problems in the newborn either by reducing antibody uptake (Ibeziako PA and Williams AI. Br. J. Obst. Gynecol., 1980, 87:976-82) or reducing in-uterus priming. The consequences of treatment with this or other drugs have not been fully explored.

Adjuvants are substances which enhance specific immune response to an antigen, causing it to be induced faster and increasing its duration (Vogel FR. Dev. Biol. Stand., 1998, 92:241-248). Alumina is still the most widely used adjuvant in parenteral vaccines. However, it is not a potent adjuvant which could help new subunit vaccines. It is only considered to have the properties of a distribution system, although there have been recent descriptions of a new toll-like receptor-independent mechanism of action involving Nalp3 (Eisenbarth SC et al. Nature, 2008, 453:1122-1126). Its mucosal use is also unproven, and it is assumed that it preferably induces a Th2 response. Licensed vaccine adjuvants (MF59, AS02, virosome, AFPL1, Proteolin, MPL, etc.) are scarce and few are for mucosal application (CT, LT, CTB, LT mutants (LT63K, LTR72), CpG, Chitosan, ISCOM and AFCo1) (Singh M and O'Hagan DT. Pharm. Res. 2002, 19(6):715-728, Pérez O et al. Immunology and Cell Biology, 2004, 82:603-610). Only CTB has been licensed for human use in a cholera vaccine.

The use of adjuvants in vaccine formulations helps to reduce the quantity of antigen required, guide the response towards a desired pattern and reduce the number of necessary doses. We rely on a platform of proteo-cochleate (AFPL1 and AFCo1) adjuvants derived from *N. meningitidis* serum group B or similar from other microorganisms for use in parenteral or mucosal vaccines. These adjuvants induce a preferential Th1 response in murines and humans through parenteral administration characterised by: IgG and anti-PL Th1 subclasses; production of IFN-y, IL-12 and TNFa; positive delayed-type hypersensitivity response and, additionally, cytotoxic T lymphocyte (CTL) induction (Pérez O et al. Infect Immun. 2001, 69(7):4502-4508; Pérez O et al. Immunology and Cell Biology. 2004, 82:603-610; Rodriguez, T et al. Vaccine 2005, 26:1312- 21; Pérez O, et al. Scand J Immunology 2007, 66:271-77). The mucosal action of AFCo1 and AFPL1 has also been demonstrated through nasal, oral, vaginal and rectal applications and their production has been extended to similar derivatives of other microorganisms. Proteoliposomes and their derivative cochleates have also been used as adjuvants as revealed in WO/2004/047805. The simultaneous application of mucosal AFCo1 and parenteral AFPL1 with the corresponding antigens induces potent responses (OCPI patent CU/P/2008/215).

The present inventors have found a solution to the problem of malaria in newborns and children of mothers infected with *P. falciparum.* Such children essentially die of anaemia caused by uncontrolled haematic cycles due to their inability to develop protective immune responses. They acknowledge the parasite derivatives which travel from an infected placenta to foetuses/newborns with immature immune systems as tolerogens. As such, parasite derivatives cannot be recognised as antigens but as individual substances or tolerogens.

The present inventors have found that, in malaria, placental necrosis allows for a greater transfer of tolerogens when the infection occurs at the end of gestation. This is due to the thinning of the placenta during the final stage of gestation which increases the effect of necrosis at the placental level to transfer the tolerogens.

The present inventors have found, in a murine specimen of *P*. *berghei* and as also caused by *P. falciparum,* that the signs of malaria (foetal and maternal death, stillborns and low birth weight) are more severe if the infection occurs at the end of gestation, i.e. close to labour.

The present inventors have found, in a gestating murine specimen infected with *P. berghei,* that anaemia is a sign of malaria in newborns and is increased by a new reinfection of newborns.

The present inventors have found that congenital malaria, which is uncommon in humans, is very common in a murine specimen of *P*. *berghei.*

The present inventors have found that parasites and/or their derivatives act as tolerogens in the immunologically immature foetal/newborn environment, resulting in a diminished anti-merozoite IgG antibody response and a primarily Th2 pattern.

The present inventors have used tolerogens to obtain formulations with proprietary adjuvants which are potent inducers of auxiliary T-lymphocyte (Th1) and cytotoxic (CTL) responses.

The present inventors have found malaria tolerogens formulated with potent adjuvants to be efficient in inducing Th1 cell responses in newborns.

The **aim of this invention** is to use tolerogens transferred at an early stage from mother to foetus/newborn and to formulate them with potent adjuvants, particularly derivatives of *Neisseria meningitidis* (AFPL1 and AFCo1), inducers of Th1 and CTL responses for use in prophylactic or therapeutic vaccines against vertically transmitted infections, especially malaria. The use of these formulations at early ages reveres the tolerance inducing protective responses.

The term "vertically transmitted" is used to describe infections, antigens or tolerogens that are transferred from the mother to the foetus/newborn through the placenta or during lactation.

The term "tolerogen" is used in a broad immunological sense and must not be interpreted in a way in which it is limited to a single component. It may refer to an epitope, a protein, a glycoprotein, a saccharide, an allergen, the total merozoite or any of their components or substances excreted/secreted by microorganisms, especially *Plasmodium.*

The term "tolerance" is used in a broad immunological sense and must not be interpreted in a way in which it is limited to the specific absence of immune response. This term may be interpreted as an absence or reduction of specific humoral response; deviation from a Th1 to a Th2 protective pattern; presence of a suppressor response in the cytokines (such as IL-10 and/or TGFB) or regulatory T-cells; absence of CTL response, and non-induction of an adequate immune memory.

The term "regulatory T" applies to natural regulatory cells (Trl) or inducible cells (Th3, Treg, etc.)

The term "Proteoliposome (PL)" is used for nanoparticles obtained from bacteria using any known method, such as: detergent-free extraction; any process which involves detergent (e.g. deoxycholate, sodium dodecyl sulfate (SDS), etc.) or extraction using vesicles ("blebs") of culture supernatants, especially those disclosed in US 5,597,572. PLs contain different pathogen-associated molecular patterns (PAMPs), molecular structures preserved among pathogens which are able to strongly stimulate the innate immune system, and particularly dendritic cells, to induce a potent adaptive response. PLs also contain protectogenic proteins, for which they are not only used as adjuvants against heterologous antigens but also as vaccines. These contain structures of the outer membrane of bacteria; but, for the purpose of this patent, those extracted from other organisms such as viruses, funguses, protozoans, helminths or tumour cells are also considered. The term AF (Adjuvant Finlay) PL is reserved for when PL is used to refer to an adjuvant.

"Cochleate (Co)" refers to a PL derivative which therefore contains various PAMPs as indicated in the patent (WO/2004/047805, Pérez O et al. Infect Immun. 2001, 69(7):4502-4508 and Pérez O et al. Immunology and cell Biology. 2004, 82:603-610). AFPL1 represents the PL extracted from serum group B of *N. meningitidis.* Also, for the purpose of this patent, the concept of Co extends to their production using combinations of synthetic lipids with the inclusion of PAMPs which act in synergy, differing radically from other Cos produced using synthetic lipids which are not included in the aforementioned patent.

It was surprising to find that, unlike in pregnant humans infected with *P. falciparum* where only a relatively low percentage of newborns develop congenital malaria, 100% of newborns of gestating murines (mice and rats) infected with *P. berghei* contained merozoites, i.e. displaying congenital malaria.

It was unexpected to reproduce many of the primary signs of human malaria (maternal death, miscarriage, anaemia, stillborns and low birth weight) in murine specimens (mice and rats).

It was surprising to find a higher frequency of malaria signs in gestating mothers infected at the end of the pregnancy in murine specimens (mice and rats), as also occurs in pregnant humans infected with *P*. *falciparum.*

It was surprising to find a greater severity of infections in younger mothers than in adults in murine specimens (mice and rats), as also occurs in pregnant humans infected with *P. falciparum.*

It was unexpected to see a reduction in Th1 immune response (production of specific IgG2a) and an increase in Th2 response (specific IgGl) in newborn mice of infected mothers.

It was surprising to find that AFPL1 and AFCo1 function in newborns and induce a memory response in murine specimens (mice and rats).

It was even more surprising to find that such formulations also work by way of the mucosa and in simultaneous (single-dose) mucosal and parenteral applications in murine specimens (mice and rats).

The **novelty** of the invention lies primarily in its demonstration that malaria during gestation produces congenital malaria in murine specimens at much higher rates than those reported for humans.

It is also new that, like in humans where young pregnant mothers are the most affected and when infections occur during the third trimester of pregnancy, but without producing congenital malaria in newborns, in gestating murines it is the young and especially those infected at the end of the pregnancy (full term) that are the most affected, except that in the latter congenital malaria is induced in the offspring.

It is also new that this infection behaves as tolerogens as the antibody response reduces and these follow a Th2 pattern (IgGl).

It is also new that formulations containing AFCo1 and AFPL1 and malaria antigens induce Th1 response.

It is also new that formulations containing AFCo1 and AFPL1 function by way of the mucosa, including in single-dose immunisations.

It is also new that formulations containing AFCo1 and AFPL1 function in newborns and also induce immune memory response at early ages.

### The proposed solution offers the following benefits:

- Placental necrosis, induced primarily but not exclusively by *P*. *falciparum,* allows for the transfer of merozoites and merozoite derivatives which induce tolerance rather than antigen stimulation in foetuses/newborns, which may be reversed by effective early immunisations.
- Formulations with the potent adjuvants AFPL1 and AFCo1 containing antigens/tolerogens of malaria reverse tolerance induced at early ages by the transfer of tolerogens from expectant mothers infected with malaria to foetuses/newborns.
- Formulations with the potent adjuvants AFPL1 and AFCo1 containing antigens/tolerogens of malaria induce a pattern of Th1 and CTL immune response.
- Formulations containing AFCo1 as an adjuvant function nasally by inducing potent systemic immune responses at the cellular (Th1) and antibody level, as well as a higher-quality immune response characterised by a response at the mucosal level.
- Formulations with the potent adjuvants AFCo1 and AFPL1 function in simultaneous mucosal and parenteral applications (single-dose vaccines).
- Formulations containing the potent adjuvants AFCo1 and AFPL1 are functional at early ages (newborns).
- Formulations containing the potent adjuvants AFCo1 and AFPL1 may be extended to protect against other vertically transmitted infections.
- Other aims and benefits of this invention shall be made apparent in the following descriptions of the invention which include figures.

### The present invention shall be described using the following specific examples.

**Example 1. Obtaining Proteoliposome (PL)**. To obtain PL, a culture is taken of any serum group of *N. meningitidis* (particularly group B), *Salmonella Typhi, Vibrio cholerae, Echerichia coli, Shiguella, Salmonella, Bordetella pertusus* or *Micobacterium tuberculosis* and the biomass collected through centrifugation is subject to a detergent, enzyme and ultrasound-based extraction process. Cell debris is removed through centrifugation and the supernatant is then digested with nucleases to eliminate nucleic acids. The extract is recovered through ultracentrifugation, re-suspended in a solution with detergent and purified of all components of a low and medium molecular weight by size-exclusion chromatography. The resulting PL of *N. meningitidis* serum group B contains: porins (PorA and PorB); traces of bacterial DNA and less than 10% (in relation to proteins) of native LPS inserted into its structure; but never free. The porins, DNA and LPS found are PAMPs which interact in synergy with the pattern recognition receptors, triggering warning signals among the cells involved in the innate response. The final product is subject to a series of biological and physiochemical tests. Where the LPS, in addition to being a PAMP, is also the antigen of interest, this can be increased to at least 35% in relation to the proteins. A similar procedure used to obtain other enriched liposomal structures in outer membrane proteins has been used based on viruses and protozoans.

**Example 2. Obtaining Cochleate (Co) derived from Proteoliposome (PL)**. The process begins with PL obtained following the methods described in EP 885900077.8 or US 5, 597,572. These are re-suspended in a Tris-EDTA buffer solution with 0.5% sodium deoxycholate. The protein concentration was determined by Lowry and modified by Peterson (Peterson GL. Analyt. Biochem. 83, 346, 1977). The content of phospholipids which make up the PL is determined by quantifying the inorganic phosphorus (Bartlett GR. J. Biol. Chem., 1959, 234, 459-465). Both the protein and phospholipid concentrations were used to establish the optimum conditions and the amount of detergents needed to form the Co. A solution is prepared with the PL adjusted to a final protein concentration of 5-6 mg/mL in a Tris-EDTA buffer containing sodium deoxycholate at a quantity of 6 to 15 times the total protein amount. This solution is filtered directly into the dialysis machine through a filter with a pore size of 0.2 µm. Dialysis is conducted by means of rotational agitation, tangential filtration, or a closed system for 24 hours with a continuous and slow change of the dialysis buffer the first time, several washes the second time and volume increments as required the third time. The final solution consisted of NaCl 50-150 mM, Imidazol 1-4 mM, HEPES 3-5 mM and CaCl 2-7 mM in H2O prepared under sterile conditions which were maintained during all of the steps. Co formation is proven by the formation of a white precipitate followed by optical and electronic microscopic observation. The protein and phospholipid concentrations are re-estimated and adjusted for later studies. The physicochemical properties of the proteins included in the Co are checked and compared with those of the PL using polyacrylamide gel electrophoresis coloured Coomassie Brilliant Blue. Their structural integrity was also checked and proven using the Western blot method.

### Example 3. Prolongation of gestation in young rats infected with Plasmodium berghei NK65 close to the birth

**Infection protoco**l: The *P. berghei* NK65 strain is maintained over successive steps in mice, and was later adapted to rats. Young or adult female Sprague-Dawley rats, 7±2 or 11 weeks from birth, respectively, were paired and their gestation was examined for mucus plug loss. They were subsequently infected with *P. berghei* NK65 at least 7, 5 or 3 days prior to the average birth date in control pregnant females (22 days). On day -3 from birth, the infection was considered as end-of-term, i.e. delayed. The blood of the infected rats was subject to microscopic examination. The rats were observed twice a day and the days of labour were determined. Labour was considered late when the rats gave birth at least one day after the control rats. The tests were repeated at least 3 times.

**Results**: Non-infected Sprague-Dawley rats used as the control group gave birth after 22 days of gestation which is typical of the breed. All pregnant rats infected at an early stage of gestation, 7 days before birth (7), also gave birth after 22 days. Contrastingly, those infected later at days -5 and -3 showed delayed labour, especially the younger rats in which the delay was 3 days. Adult rats were delayed by just one day (Table 1).

**Table 1. Prolongation of gestation in young rats infected close to birth**

| **Gestation** | **Day of infection** | **Delay (days)** |
|---|---|---|
| **Young** | **-3** | **3** |
| | **-5** | **3** |
| | **-7** | **0** |
| **Adults** | **-3** | **1** |
| | **-5** | **1** |
| | **-7** | **0** |

**Summary:** Infection with *P. berghei* at a late stage of pregnancy produces delayed birth, which is more accentuated in younger pregnant females.

### Example 4. Stillborns in young rats infected close to birth and neonatal death in adult rats infected close to birth

**Infection protocol:** Young or adult female Sprague-Dawley rats, 7±2 or 11 weeks from birth, respectively, were paired and their gestation was examined for mucus plug loss. The rats were subsequently infected with *P. berghei* NK65 at 7, 5 or 3 days prior to birth. On day -3 from birth, the infection was considered as end-of-term, or delayed. The blood of the infected rats was subject to microscopic examination. The rats were observed at least 4 times a day and the percentages of stillborns and neonatal death were determined. These were quantified from post-partum days 1 to 5. The tests were repeated at least 3 times.

**Results:** In the non-infected Sprague-Dawley rats used as the control group there were no observations of stillborns or neonatal death. In pregnant rats infected at an early stage (7 or 5 days before birth), there were no observations of stillborns. Contrastingly, pregnant rats infected end-of-term (3 days before birth) produced stillborns in both adults (12.5%) and young (75%). However, the death rate was much higher in the young. Neonatal death occurred in adult rats infected at end-of-term (3 days before birth). The high birth rate explains why death did not occur in newborns of young rats infected end-of-term (Table 2).

**Table 2. Stillborns in young rats infected close to birth and neonatal death in adult rats infected close to birth**

| **Gestation** | **Day of infection** | **Stillborns (%)** | **Neonatal death (%)** |
|---|---|---|---|
| **Young** | **-3** | **75** | **0** |
| | **-5** | **0** | **0** |
| | **-7** | **0** | **0** |
| **Adults** | **-3** | **12.5** | **43** |
| | **-5** | **0** | **0** |
| | **-7** | **0** | **0** |

**Summary:** Infection with *P. berghei* at a late stage of pregnancy produces a high death rate, especially in young pregnant female rats, as well as neonatal death.

### Example 5. Young rats infected end-of-term had newborns with a low birth weight

**Infection protocol:** Young or adult female Sprague-Dawley rats, 7±2 or 11 weeks from birth, respectively, were paired and their gestation was examined for mucus plug loss. The rats were subsequently infected with *P. berghei* NK65 at 7, 5 or 3 days prior to birth. On day -3 from birth, the infection was considered as end-of-term, or delayed. The blood of the infected rats was subject to microscopic examination. The rats were observed at least 4 times a day and the newborn weight in grams (g) of surviving mice was measured. The tests were repeated at least 3 times.

**Results:** In the non-infected Sprague-Dawley rats used as the control group, newborn weight was higher among young pregnant females than in adults. The infection in adult pregnant females did not alter the weight of their newborns in any of the cases. Contrastingly, in young pregnant rats infected end-of-term (3 days before birth) the newborn weight was reduced (Table 3). **Table 3. Young rats infected end-of-term had newborns with a low birth weight**

| **Gestation** | **Day of infection** | **Surviving newborn weight ± ED (g)** |
|---|---|---|
| **Young** | **-3** | **5.26 ±0.45** |
| | **-5** | **7.23 ±0.83** |
| | **-7** | **8.79 ±0.65** |
| **Control** | | **8.18 ±0.46** |
| **Adults** | **-3** | **5.96 ±0.89** |
| | **-5** | **5.88± 0.56** |
| | **-7** | **5.48 ±0.32** |
| **Control** | | **6.67 ±0.65** |

**Summary:** End-of-term infection with *P. berghei* NK65 during pregnancy reduces the weight of newborns.

### Example 6. Young rats infected end-of-term showed maternal death

**Infection protocol:** Young or adult female Sprague-Dawley rats, 7±2 or 11 weeks from birth, respectively, were paired and their gestation was examined for mucus plug loss. The rats were subsequently infected with *P. berghei* NK65 at 7, 5 or 3 days prior to birth. On day -3 from birth, the infection was considered as end-of-term, or delayed. The blood of the infected rats was subject to microscopic examination. The rats were observed at least 4 times a day and the rate of maternal death was recorded. The tests were repeated at least 3 times.

**Results:** In the non-infected Sprague-Dawley rats used as the control group, and in adult pregnant rats infected on different days, no cases of maternal death were observed. Maternal death was also absent in young pregnant females infected on days -7 or -5. Contrastingly, in young pregnant rats infected end-of-term (3 days before birth), maternal death was observed (Table 4).

**Table 4. Young rats infected end-of-term showed maternal death**

| **Gestation** | **Day of infection** | **Maternal death** |
|---|---|---|
| **Young** | **-3** | **5/12** |
| | **-5** | **0/12** |
| | **-7** | **0/12** |
| **Control** | - | **0/12** |
| **Adults** | **-3** | **0/12** |
| | **-5** | **0/12** |
| | **-7** | **0/12** |
| **Control** | - | **0/12** |

Summary: Infection with *P. berghei* at a late stage of pregnancy produces a higher rate of maternal death in young pregnant females.

### Example 7. Congenital malaria in newborns of infected mothers

**Infection protocol:** Female Sprague-Dawley rats were paired and their gestation was examined for mucus plug loss. The rats were subsequently infected with *P. berghei* NK65 at 7, 5 or 3 days prior to birth. In both the rats and their offspring, the infection was confirmed by microscopic examination of their blood. The tests were repeated at least 3 times.

**Results:** All newborns of mothers infected at any stage of gestation acquired congenital malaria, i.e. merozoites were found in their blood.

**Summary:** 100% of the newborns of infected mothers acquired congenital malaria.

### Example 8. Presence of anaemia in newborns of infected mothers

**Infection protocol:** Female Sprague-Dawley rats were paired and their gestation was examined for mucus plug loss. The rats were subsequently infected with *P. berghei* NK65 at 7, 5 or 3 days prior to birth. In the rats, the infection was confirmed by microscopic examination of their blood. The newborns of infected or healthy mothers were also infected with *P. berghei* NK65 at 5 days prior to birth via intraperitoneal injection. A group deriving from infected mothers was not infected and was used as a control group. Anaemia was measured as a percentage. The tests were repeated at least 3 times.

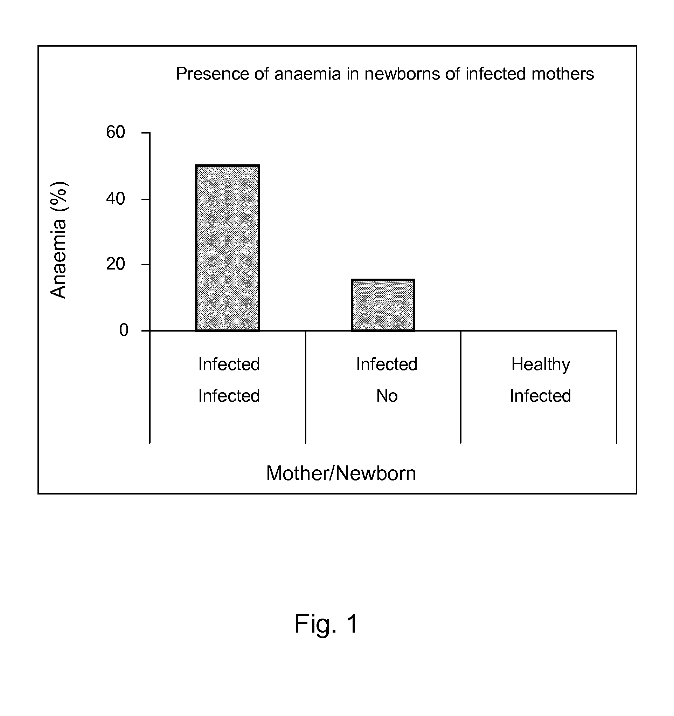

**Results:** Anaemia was observed in 18% of the offspring of mothers infected with *P. berghei* NK65, which rose to 50% when the newborns were re-infected with the same strain (Figure 1).

**Summary:** Congenital malaria induces anaemia in newborns and is increased by re-infection.

### Example 9. Potent Th1 response induced by formulations of malaria peptides and Finlay Adjuvants administered via intramuscular or intranasal administration in newborns with memory induction.

**Protocol:** *P. falciparum* peptides were formulated with AFPL1 or AFCo1. Balb/c mice 7 days post-partum received intramuscular immunisation of AFPL1+peptide in 2 intramuscular doses administered 14 days apart; AFCo1+peptide in 3 intranasal doses administered 7 days apart, or a simultaneous application via both forms of administration. The response in saliva and in anti-peptide serum was evaluated 7 and 21 days after the last dose. A booster shot was applied 3 months after immunisation to evaluate induced immune memory.

**Method of blood sampling and processing used to obtain serum:** Blood was drawn 28 days post-partum through a small notch in the tail. The blood samples taken were incubated for 1 hr at 37°C and then centrifuged at 2000 g for 10 min to extract the serum.

### ELISA detection of anti-merozoite IgG:

### Reagents and Solutions:

Coating buffer solution (CBS): Na₂C0₃ 11 mM, NaHC0₃ 35 mM (pH 9.6)

Phosphate buffered saline solution (PBSS) 0.15 M (pH 7.2)

Blocking solution (PBSS / BSA 1%): PBSS 0.15 M (pH 7.2), Bovine Serum Albumin at 1% (w/v)

Washing solution (PBSS, Tween 20 at 0.1%): PBSS, Tween 20 at 0.1% (v/v), pH 7.4)

Sample dilution solution (PBSS, BSA 1%, Tween 20 at 0.1%)

Peroxidase conjugated anti-mouse IgG (Sigma, St. Louis, MO, USA)

Substrate buffer solution, SBS (Na₂HP0₄ 52 mM and citric acid 25 mM (pH 5.6))

Stopper solution: H₂SO₄ 2 M.

### Methodology:

- 100 µL/well of merozoite antigens purified to a concentration of 10 µg/mL and diluted in CBS is added to high binding capacity 96 well plates (Maxisorp, Nunc, EUA) and incubated overnight at 4°C in a humidity chamber
- Three washes are performed with PBSS
- 100 µL/well of blocking solution is added and it is incubated for 1 hr at room temperature in a humidity chamber
- Three washes are performed with washing solution
- 100 µL/well in duplicate dilutions of the individual serums are applied and incubated for 2 hrs at 37°C
- Three washes are performed with washing solution
- 100 µL/well of anti-IgG conjugate diluted to 1:2000 is added to a diluted solution of the samples and incubated for 1 hr at 37°C in a humidity chamber
- Three washes are performed with washing solution
- 100 µL/well of a hydrogen peroxide 0.01% (v/v) and of the chromogene o-phenylenediamine (OPD) 0.6 mg/mL solution in SBS is added and incubated in the dark for 30 min
- The reaction is stopped with the addition of 50 µL of a stopper solution. Optical density (OD) absorbance is measured at 492 nm using a microplate reader (Titertek, Multiskan Plus)

**Saliva sampling and processing method:** saliva was sampled 7 days after administration of the last dose. To draw saliva samples, salivation was stimulated by an intraperitoneal application of 50 µL per mouse of pilocarpine 0.5% (Imefa, Cuba). The samples were kept on ice during extraction and were immediately inactivated at 56°C for 15 min. They were then centrifuged at 14000 g for 15 min at 4°C, after which the supernatant was collected and stored at -70°C until use.

### ELISA detection of anti-peptide IgA:

### Reagents and Solutions:

Coating buffer solution (CBS): Na₂C0₃ 11 mM, NaHC0₃ 35 mM (pH 9.6)

Phosphate buffered saline solution (PBSS) 0.15 M (pH 7.2)

Blocking solution (PBSS / BSA 1%): PBSS 0.15 M (pH 7.2), Bovine Serum Albumin at 1% (w/v)

Washing solution (PBSS, Tween 20 at 0.1%): PBSS, Tween 20 at 0.1% (v/v), pH 7.4)

Sample dilution solution (PBSS, BSA 1%, Tween 20 at 0.1%)

Biotin peroxidase conjugated anti-mouse IgA (R5-140) (Sigma, St. Louis, MO, USA)

Streptavidin-peroxidase (Sigma, St. Louis, MO, USA)

Substrate buffer solution, SBS (Na₂HP0₄ 52 mM and citric acid 25 mM (pH 5.6))

Stopper solution: H₂SO₄ 2 M.

Standard antibody saliva: As an internal antibody standard, for anti-PL IgA tests, a curve comprising hyperimmune mouse saliva samples was used.

### Methodology:

- 100 µL/well of peptide at a concentration of 10 µg/mL and diluted in CBS is added to high binding capacity 96 well plates (Maxisorp, Nunc, EUA) and incubated overnight at 4°C in a humidity chamber
- Three washes are performed with PBSS
- 100 µL/well of blocking solution is added and it is incubated for 1 hr at room temperature in a humidity chamber
- Three washes are performed with washing solution
- The standard antibody saliva and the salivas to be tested are diluted 1:2 in sample dilution solution. 100 µL/well in duplicate dilutions of the individual serums and of the reference serum are applied and incubated for 2 hrs at 37°C
- Three washes are performed with washing solution
- 100 µL/well of anti-IgA conjugate at a concentration of 2 µg/mL is added to a diluted solution of the samples and incubated for 2 hr at 37°C in a humidity chamber
- Three washes are performed with washing solution
- A second conjugate (streptavidin-peroxidase) at a dilution of 1:2000 is then added to the diluted sample solution and incubated for 30 min at 37°C
- Five washes are performed with washing solution
- 100 µL/well of a hydrogen peroxide 0.01% (v/v) and of the chromogene o-phenylenediamine (OPD) 0.6 mg/mL solution in SBS is added and incubated in the dark for 30 min
- The reaction is stopped with the addition of 50 µL of a stopper solution
- Optical density (OD) absorbance is measured at a reading of 492 nm on a microplate reader (Titertek, Multiskan Plus)

**Results:** A systemic anti-peptide IgG response was induced with both forms of immunisation and a specific IgA response was induced in saliva with nasal immunisation. Simultaneous application via both forms of administration also induced systemic and mucosal response. A dose at 3 months post-partum (memory) increased both immune responses.

**Summary:** Adjuvant formulations induce systemic and mucosal immune and memory responses in newborn mice.

### Example 10. Reduced anti-merozoite IgG in newborns of mothers infected with P. berghei

**Infection protocol:** Female Balb/c mice were paired and their gestation was examined for mucus plug loss. They were subsequently infected with *P. berghei* NK65 at 3 days prior to birth. In both the gestating mice and their offspring, infection was confirmed by microscopic examination of their blood. A group of gestating mothers was not infected and their offspring were used as a control group. These were infected with *P. berghei* NK65 at 5 days post-partum via intraperitoneal administration. Blood samples were taken from the newborns from which the serum was extracted. In these mice the induced anti-merozoite IgG response was evaluated with ELISA. The tests were repeated at least 3 times.

Anti-peptide IgG response was evaluated as described in example 9.

**Results:** The newborns of infected mothers showed a lower anti-merozoite IgG response than infected newborns of healthy mothers.

**Summary:** The newborns of infected mothers show less recognition of *P. berghei* infection than infected healthy newborns, i.e. tolerance.

### Example 11. Alteration of the recognition pattern in anti-merozoite IgG subclasses in newborn mice of mothers infected with P. berghei

**Infection protocol:** Since with mice the Th1/Th2 patterns are clearer in subclasses, experiments to measure these were performed using female Balb/c mice. The specimens were paired and their gestation was examined for mucus plug loss. They were subsequently infected with P. *berghei* NK65 at 3 days prior to birth. In both the gestating mice and their offspring, infection was confirmed by microscopic examination of their blood. A group of gestating mothers was not infected and their offspring were used as a control group. These were infected with *P. berghei* NK65 at 5 days post-partum via intraperitoneal administration. Blood samples were taken from the newborns from which the serum was extracted. In these mice the induced anti-merozoite IgG1 to IgG2a response was evaluated with ELISA. The tests were repeated at least 3 times.

**Method of blood** sampling and processing used to obtain **serum:** The procedure described in example 9 was followed.

### Detection of anti-merozoite IgG1 and IgG2a subclasses using ELISA:

### Reagents and Solutions:

Coating buffer solution (CBS): Na₂C0₃ 11 mM, NaHC0₃ 35 mM (pH 9.6)

### Phosphate buffered saline solution (PBSS) 0.15 M (pH 7.2)

Blocking solution (PBSS / BSA 1%): PBSS 0.15 M (pH 7.2), Bovine Serum Albumin at 1% (w/v)

Washing solution (PBSS, Tween 20 at 0.1%): PBSS, Tween 20 at 0.1% (v/v), pH 7.4)

Sample dilution solution (PBSS, BSA 1%, Tween 20 at 0.1%)

Biotinylated goat anti-mouse IgG1 and IgG2 AcM (Amersham, LIFE SCIENCES)

Streptavidin-peroxidase (Sigma, St. Louis, MO, USA)

Substrate buffer solution, SBS (Na₂HP0₄ 52 mM and citric acid 25 mM (pH 5.6))

Stopper solution: H₂SO₄ 2 M.

### Methodology:

- 100 µL/well of PL at a concentration of 20 µg/mL and diluted in CBS is added to high binding capacity 96 well plates (Maxisorp, Nunc, EUA) and incubated overnight at 4°C in a humidity chamber
- Three washes are performed with PBSS
- 100 µL/well of blocking solution is added and it is incubated for 1 hr at room temperature in a humidity chamber
- Three washes are performed with washing solution
- The serums to be tested are diluted 1:100 in sample dilution solution. 100 µL/well in duplicate dilutions of the individual serums and of the reference serum are applied and incubated for 2 hrs at 37°C
- Three washes are performed with washing solution
- 100 µL/well of anti-mouse IgG1 or IgG2a conjugate diluted to 1:2000 is added to a diluted solution of the samples and incubated for 2 hr at 37°C in a humidity chamber
- Three washes are performed with washing solution
- A second conjugate (streptavidin-peroxidase) at a dilution of 1:2000 is then added to the diluted sample solution and incubated for 30 min at 37°C
- Five washes are performed with washing solution
- 100 µL/well of a hydrogen peroxide 0.01% (v/v) and o-phenylenediamine (OPD) 0.6 mg/mL solution in SBS is added and incubated in the dark for 30 min
- The reaction is stopped with the addition of 50 µL of a stopper solution
- Optical density (OD) absorbance is measured at a reading of 492 nm on a microplate reader (Titertek, Multiskan Plus)

The results for anti-PL IgGl and IgG2a were expressed in optical density (OD). In this test, samples with a DO higher than 0.25 are considered positive.

Results: In newborns of infected mothers the IgGl (Th2) response was predominant, while an anti-merozoite IgG2a (Th1) response was also detected in the offspring of healthy mothers that were infected after birth.

**Summary:** An alteration in anti-malaria Th1 response was found in the offspring of infected mothers.

### Example 12. Induction of Th1 response with formulations of Plasmodium berghei merozoite antigens formulated with potent adjuvants revert tolerance in newborns of infected mothers

**Infection protocol:** Balb/c mice 10 days post-partum (newborns) from infected mothers were immunised with formulations of AFPL1+*P*. *berghei* antigens or AFCo1*+P. berghei* antigens applied in two doses administered 14 days apart. A control group from healthy mothers was also used. The infection was confirmed by microscopic examination of their blood before the immunisations were administered. Blood samples were taken from the newborns 21 days after administration of the last dose and the serum was extracted. In these mice the induced anti-P.falciparum antigen IgG1 to IgG2a response was evaluated with ELISA. The tests were repeated at least 3 times.

**Results:** The control mice from non-infected mothers induced a IgG2a response to *P. berghei* merozoites. Likewise, the mice from infected mothers also induced a Th1 (IgG2a) pattern.

**Summary:** Formulations with adjuvanted antigens were able to revers the tolerance of newborns from infected mothers.

## Claims

1. Vaccine formulations for immunising mammals against tolerogens with potent adjuvants which evoke cellular (Th1 and CTL) immune responses and their corresponding antibodies which break tolerance induced by the vertical transmission of tolerogens through parenteral and/or mucosal administration.

2. Vaccine formulations as described in claim 1, where the tolerogen derives from malaria, an allergen or another vertically transmitted infection.

3. Vaccine formulations as described in claim 2, where malaria is produced by the *Plasmodium* genus, particularly *P. falciparum* or *P. vivax.*

4. Vaccine formulations as described in claim 3, where the tolerogen is the merozoite or one of its constituents or derivatives.

5. Vaccine formulations as described in claim 4, where the constituent is a protein, a phospholipid, a saccharide or any combination thereof.

6. Vaccine formulations as described in claim 2, where the allergen derives from a mite, pollen or animal derivative in a natural or recombinant form.

7. Vaccine formulations as described in claim 1, where the adjuvant is AFCo1, AFPL1 or another adjuvant which induces Th1 and CTL immune response or functions in the mucosa.

8. Use of vaccine formulations which involve simultaneous or independent mucosal or parenteral administration.

9. Treatment method **characterised in that** it includes the application of vaccine formulations which contain tolerogenic adjuvants.
